# EUROPEAN PATENT APPLICATION

(11) **EP 1 449 925 A1**
(43) Date of publication of application: **25.08.2004**
(21) Application number: 03290412.0
(22) Date of filing: 20.02.2003
(51) Int. Cl.: C12Q 1/68, C12N 15/12, C07K 14/47, C07K 16/18, G01N 33/50, A61K 48/00, A01K 67/027

(54) **Method of diagnosis of type 2 diabetes**

(71) Applicant: CENTRE NATIONAL DE LA RECHERCHE SCIENTIFIQUE (CNRS), 75016 Paris (FR)
(72) Inventor: Froguel, Philippe, 93170 Bagnolet (FR); Dina, Christian, 75009 Paris (FR); Melloul, Danielle, Ashkelon (IL); Neve, Bernadette Paulina, 8930 Menen (BE)
(74) Representative: Warcoin, Jacques

(57) **Abstract**

The invention pertains to the field of human genetics and relates to new methods of diagnosis and therapy of type 2 diabetes, based on the identification of the A185G variation located at exon 2 of the *tieg2* gene as well as the G-1534C, 54a and 304a variations.

SEQUENCE LISTING

<110> CENTRE NATIONAL DE LA RECHERCHE SCIENTIFIQUE (CNRS)

<120> METHOD OF DIAGNOSIS OF TYPE 2 DIABETES

<130> D20471

<140>
<141>

<160> 39

<170> PatentIn Ver. 2.1

<210> 1
<211> 1539
<212> DNA
<213> Homo sapiens

<220>
<221> CDS
<222> (1)..(1539)
<223> Tieg2wt

<400> 1
atg cac acg ccg gac ttc gca ggc cca gac gac gcg cgc gca gtt gac    48
Met His Thr Pro Asp Phe Ala Gly Pro Asp Asp Ala Arg Ala Val Asp
  1               5                  10                  15

atc atg gac ata tgt gag tcc atc ctg gag agg aag cgg cat gac agc    96
Ile Met Asp Ile Cys Glu Ser Ile Leu Glu Arg Lys Arg His Asp Ser
             20                  25                  30

gaa agg tct act tgc agc atc ttg gag cag aca gac atg gaa gct gtc    144
Glu Arg Ser Thr Cys Ser Ile Leu Glu Gln Thr Asp Met Glu Ala Val
         35                  40                  45

gag gct ctt gtt tgt atg agc tcc tgg ggt caa aga tcc cag aaa ggt    192
Glu Ala Leu Val Cys Met Ser Ser Trp Gly Gln Arg Ser Gln Lys Gly
     50                  55                  60

gac ctg ttg cgg ata aga ccc ctc acg cct gtc tct gac tct ggg gat    240
Asp Leu Leu Arg Ile Arg Pro Leu Thr Pro Val Ser Asp Ser Gly Asp
 65                  70                  75                  80

gtc acc acc act gtg cat atg gat gca gcc aca cct gaa cta cca aaa    288
Val Thr Thr Thr Val His Met Asp Ala Ala Thr Pro Glu Leu Pro Lys
                 85                  90                  95

gac ttc cat tct tta tcg act ctg tgc ata act cct cct cag agc cct    336
Asp Phe His Ser Leu Ser Thr Leu Cys Ile Thr Pro Pro Gln Ser Pro
            100                 105                 110

gat ctc gtg gag cca tcg aca agg aca cct gtt tct ccc caa gta aca    384
Asp Leu Val Glu Pro Ser Thr Arg Thr Pro Val Ser Pro Gln Val Thr
        115                 120                 125

gat tcc aaa gca tgt aca gcc acg gat gtt ctc cag tcc tct gcc gta    432
Asp Ser Lys Ala Cys Thr Ala Thr Asp Val Leu Gln Ser Ser Ala Val
    130                 135                 140

gtg gcc aga gct ctg agc ggg ggc gcg gag agg ggc ttg ctg ggt ttg     480
Val Ala Arg Ala Leu Ser Gly Gly Ala Glu Arg Gly Leu Leu Gly Leu
145                 150                 155                 160

gag cca gtg ccc agc tct ccc tgc agg gcc aag ggg act agc gtg atc     528
Glu Pro Val Pro Ser Ser Pro Cys Arg Ala Lys Gly Thr Ser Val Ile
                165                 170                 175

cga cac act ggg gag agc cct gct gcc tgc ttt ccc acc atc cag act     576
Arg His Thr Gly Glu Ser Pro Ala Ala Cys Phe Pro Thr Ile Gln Thr
            180                 185                 190

cca gat tgc cgg ctt tct gac agc aga gaa gga gaa gag cag ctt ctg     624
Pro Asp Cys Arg Leu Ser Asp Ser Arg Glu Gly Glu Glu Gln Leu Leu
        195                 200                 205

gga cac ttt gaa act ttg cag gac aca cac ctc acg gac agt tta ctc     672
Gly His Phe Glu Thr Leu Gln Asp Thr His Leu Thr Asp Ser Leu Leu
    210                 215                 220

agc act aac ttg gtg tcc tgt cag ccc tgc ttg cac aag tct ggt ggc     720
Ser Thr Asn Leu Val Ser Cys Gln Pro Cys Leu His Lys Ser Gly Gly
225                 230                 235                 240

ctg ctg ctc act gac aaa ggc cag cag gca ggg tgg cct ggt gca gtt     768
Leu Leu Leu Thr Asp Lys Gly Gln Gln Ala Gly Trp Pro Gly Ala Val
                245                 250                 255

cag act tgc tca cca aag aat tat gaa aat gac ctg ccc agg aaa acc     816
Gln Thr Cys Ser Pro Lys Asn Tyr Glu Asn Asp Leu Pro Arg Lys Thr

            260                 265                 270

acc cct ctg att tct gtc tct gtc cct gct ccc cct gtc ctt tgc cag     864
Thr Pro Leu Ile Ser Val Ser Val Pro Ala Pro Pro Val Leu Cys Gln
        275                 280                 285

atg atc cct gtg act gga caa agt agc atg tta cca gct ttt ttg aag     912
Met Ile Pro Val Thr Gly Gln Ser Ser Met Leu Pro Ala Phe Leu Lys
    290                 295                 300

ccc cct ccc cag ttg tct gtg ggg act gtg aga ccc atc cta gct cag     960
Pro Pro Pro Gln Leu Ser Val Gly Thr Val Arg Pro Ile Leu Ala Gln
305                 310                 315                 320

gct gct cca gcg cct caa cct gtg ttc gtg gga cct gct gtg cct cag     1008
Ala Ala Pro Ala Pro Gln Pro Val Phe Val Gly Pro Ala Val Pro Gln
                325                 330                 335

gga gct gtg atg ttg gtc ctg ccc cag gga gcc ctc cct ccg cct gcc     1056
Gly Ala Val Met Leu Val Leu Pro Gln Gly Ala Leu Pro Pro Pro Ala
            340                 345                 350

ccc tgt gca gcc aat gtc atg gct gcc ggg aat acc aag ttg ttg ccc     1104
Pro Cys Ala Ala Asn Val Met Ala Ala Gly Asn Thr Lys Leu Leu Pro
        355                 360                 365

ctt gcc cct gct cca gtg ttc atc acc tct agc caa aac tgt gtc cct     1152
Leu Ala Pro Ala Pro Val Phe Ile Thr Ser Ser Gln Asn Cys Val Pro
    370                 375                 380

cag gta gac ttt tcc cga agg agg aac tat gtt tgc agc ttc cca ggt 1200
Gln Val Asp Phe Ser Arg Arg Arg Asn Tyr Val Cys Ser Phe Pro Gly
385                 390                 395                 400

tgc cgg aag acc tac ttc aaa agt tcc cac ctt aag gcc cat ctt cgc 1248
Cys Arg Lys Thr Tyr Phe Lys Ser Ser His Leu Lys Ala His Leu Arg
                405                 410                 415

act cac aca ggg gag aag cct ttc aac tgc agc tgg gat ggc tgt gat 1296
Thr His Thr Gly Glu Lys Pro Phe Asn Cys Ser Trp Asp Gly Cys Asp
            420                 425                 430

aaa aag ttt gct cgt tcg gat gag ctg tca cgc cac cgc aga act cac 1344
Lys Lys Phe Ala Arg Ser Asp Glu Leu Ser Arg His Arg Arg Thr His
        435                 440                 445

aca ggg gag aag aag ttt gtg tgc ccg gtg tgt gac cga cgt ttc atg 1392
Thr Gly Glu Lys Lys Phe Val Cys Pro Val Cys Asp Arg Arg Phe Met
    450                 455                 460

cgc agt gac cac ctg acg aag cat gcc cgg cgc cac atg acg acc aag 1440
Arg Ser Asp His Leu Thr Lys His Ala Arg Arg His Met Thr Thr Lys
465                 470                 475                 480

aag atc cca ggc tgg cag gca gag gtt ggc aag ctg aac aga atc gcc 1488
Lys Ile Pro Gly Trp Gln Ala Glu Val Gly Lys Leu Asn Arg Ile Ala
                485                 490                 495

tct gca gag agc ccg ggg agc cca ctg gtg agc atg cca gcc tct gcc 1536
Ser Ala Glu Ser Pro Gly Ser Pro Leu Val Ser Met Pro Ala Ser Ala
            500                 505                 510

tga 1539

<210> 2
<211> 267
<212> DNA
<213> Homo sapiens

<220>
<223> Exon 2 tieg2 gene

<400> 2
gttgacatca tggacatatg tgagtccatc ctggagagga agcggcatga cagcgaaagg 60
tctacttgca gcatcttgga gcagacagac atggaagctg tcgaggctct tgtttgtatg 120
agctcctggg gtcaaagatc ccagaaaggt gacctgttgc ggataagacc cctcacgcct 180
gtctctgact ctggggatgt caccaccact gtgcatatgg atgcagccac acctgaacta 240
ccaaaagact tccattcttt atcgact 267

<210> 3
<211> 512
<212> PRT
<213> Homo sapiens

<220>
<223> TIEG2 Q62R

<400> 3
Met His Thr Pro Asp Phe Ala Gly Pro Asp Asp Ala Arg Ala Val Asp
1               5                   10                  15

Ile Met Asp Ile Cys Glu Ser Ile Leu Glu Arg Lys Arg His Asp Ser
            20                  25                  30

Glu Arg Ser Thr Cys Ser Ile Leu Glu Gln Thr Asp Met Glu Ala Val
        35                  40                  45

Glu Ala Leu Val Cys Met Ser Ser Trp Gly Gln Arg Ser Arg Lys Gly
    50                  55                  60

Asp Leu Leu Arg Ile Arg Pro Leu Thr Pro Val Ser Asp Ser Gly Asp
65                  70                  75                  80

Val Thr Thr Thr Val His Met Asp Ala Ala Thr Pro Glu Leu Pro Lys
                85                  90                  95

Asp Phe His Ser Leu Ser Thr Leu Cys Ile Thr Pro Pro Gln Ser Pro
            100                 105                 110

Asp Leu Val Glu Pro Ser Thr Arg Thr Pro Val Ser Pro Gln Val Thr
        115                 120                 125

Asp Ser Lys Ala Cys Thr Ala Thr Asp Val Leu Gln Ser Ser Ala Val
    130                 135                 140

Val Ala Arg Ala Leu Ser Gly Gly Ala Glu Arg Gly Leu Leu Gly Leu
145                 150                 155                 160

Glu Pro Val Pro Ser Ser Pro Cys Arg Ala Lys Gly Thr Ser Val Ile
                165                 170                 175

Arg His Thr Gly Glu Ser Pro Ala Ala Cys Phe Pro Thr Ile Gln Thr
            180                 185                 190

Pro Asp Cys Arg Leu Ser Asp Ser Arg Glu Gly Glu Glu Gln Leu Leu
        195                 200                 205

Gly His Phe Glu Thr Leu Gln Asp Thr His Leu Thr Asp Ser Leu Leu
    210                 215                 220

Ser Thr Asn Leu Val Ser Cys Gln Pro Cys Leu His Lys Ser Gly Gly
225                 230                 235                 240

Leu Leu Leu Thr Asp Lys Gly Gln Gln Ala Gly Trp Pro Gly Ala Val
                245                 250                 255

Gln Thr Cys Ser Pro Lys Asn Tyr Glu Asn Asp Leu Pro Arg Lys Thr
            260                 265                 270

Thr Pro Leu Ile Ser Val Ser Val Pro Ala Pro Pro Val Leu Cys Gln
        275                 280                 285

Met Ile Pro Val Thr Gly Gln Ser Ser Met Leu Pro Ala Phe Leu Lys
    290                 295                 300

Pro Pro Pro Gln Leu Ser Val Gly Thr Val Arg Pro Ile Leu Ala Gln
305                 310                 315                 320

Ala Ala Pro Ala Pro Gln Pro Val Phe Val Gly Pro Ala Val Pro Gln
                325                 330                 335

Gly Ala Val Met Leu Val Leu Pro Gln Gly Ala Leu Pro Pro Pro Ala
            340                 345                 350

Pro Cys Ala Ala Asn Val Met Ala Ala Gly Asn Thr Lys Leu Leu Pro
        355                 360                 365

Leu Ala Pro Ala Pro Val Phe Ile Thr Ser Ser Gln Asn Cys Val Pro
    370                 375                 380

Gln Val Asp Phe Ser Arg Arg Arg Asn Tyr Val Cys Ser Phe Pro Gly
385                 390                 395                 400

Cys Arg Lys Thr Tyr Phe Lys Ser Ser His Leu Lys Ala His Leu Arg
                405                 410                 415

Thr His Thr Gly Glu Lys Pro Phe Asn Cys Ser Trp Asp Gly Cys Asp
            420                 425                 430

Lys Lys Phe Ala Arg Ser Asp Glu Leu Ser Arg His Arg Arg Thr His
        435                 440                 445

Thr Gly Glu Lys Lys Phe Val Cys Pro Val Cys Asp Arg Arg Phe Met
    450                 455                 460

Arg Ser Asp His Leu Thr Lys His Ala Arg Arg His Met Thr Thr Lys
465                 470                 475                 480

Lys Ile Pro Gly Trp Gln Ala Glu Val Gly Lys Leu Asn Arg Ile Ala
                485                 490                 495

Ser Ala Glu Ser Pro Gly Ser Pro Leu Val Ser Met Pro Ala Ser Ala
            500                 505                 510


<210> 4
<211> 2818
<212> DNA
<213> Homo sapiens

<220>
<223> -1.8 kb of TIEG2

<400> 4
   gactggaggt cggccccgag ggtgtgtccg ctagaggggg cctctccagg tggactgcgg 60
   gtgagagtaa agggggcatg tgggggaaaa gcattctagg gaacagcaag tgcgaggacc 120
   tggagacggc agaggggctg gtgggacagg cgcccaagag gaggcccagg ctgtccctcc 180
   ggggcgtggg gagaggcatg gggctgggtc cctgcaggag ctcacaagcc acagtgggga 240
   gcagaagcgt tacgctgagt acagtgggag ccactgaaag ggttaaagac ccctgggggt 300
   gctgagtggg aagaggctct gggagctgga gtggacgcag ggagaccagt tataaggcga 360
   atgcaggagt ccaggcaaaa gagaatattc cagtgtgtat cttaagactt aactttttac 420
   acacagaaca ccctgcgccc ctctcagagc ctccggggtc ttcatgcgag cagtttatca 480
   cggttctttc gtcttaaacc ctcttactat ttcagcaaac accctctcct cagcctggtc 540
   aacccctctg cccaacccga gtgggccccg aaggaacaag tggggaccag agtcctgctc 600
   atgtgagtgg tggggcccag cccgttcccc taagaccaga attctctcgc tcagagcaaa 660
   gccccggggt gccgggattc tctctgctcc cgctccctgg atggagcttt taaaaaagga 720
   ggattcctca ctgcggcctc tgccccttgc actgagcgcc aacccgcatc acagctcagc 780
   aaaggcgcgc gcagtgaaca gacggaggag gggtggctcc tcgctgagct gaaggctgcg 840
   gcgacgaaaa taaacagcat ggttcctgct ctccacgaac ctggcgctgc agacgtgggc 900
   aaaccacacc aagagacgcg agcaaccgaa atatagggag aaaaaaaaag gtgccctgga 960
   cagcgggcta gatgtctcct gcaggctcga ggacttcccg gagggggcgg cgttttctcg 1020
   gggcctggga agaatggcgg ggtttcagcg ggcgccttct gggtgactgc ccgtttcggc 1080
   ttcccctgac cccgtaacag agcggaagag gcccagagac gccagaggag gggcgtcagc 1140
   gggacactca ggccaataag ctctgactcc cgctgtctgc aaaaatgacc ggatgcagga 1200
   atgtcaccta gaaccggcca gctttccgac ctctccggtc aaggacgcat tcctaagaga 1260
   cgaagggaga atgacagaag ttaaaaacaa ccaaacaaaa gccaggttga ggcctctagg 1320
   tgggtctccg ctaccgatgg attcagccgg tggggctgca ggaggttcct ataagcgtgg 1380
   tcccctctcc ccgcaccgag tccggccccg gccctgggat ttccccagcc gccgccccgc 1440
   cccctccgcc ccgccgcccc cgccgccccg ccgccccctc cgcccccgcc gccccctccg 1500
   cccccgccgc cccctccgcc ccgcttgccc ctggcgccgc tgcgaccttt cccttacttt 1560
   ccagggcctc aggccccgcc cctcgctccg accaaaacac gtgccgcgtc ccgccctgcc 1620
   cgatcacgcc gcggccaggc tccgatcacg cggccccccg cggcgctcat tggccggccg 1680
   ggcacgaatt ggctggtggt ggccggcccc gccccggcag gggcgcggtg tattttggtt 1740
   cgcctccgcg ccccgccccg ccagccgctc cctccgcggc cgccccgccc ctcccgcgcc 1800
   gcgagggccg cgccggggca gagccgcgcg ggcgggcgag gcgcgtgccg gccgcaggag 1860
   ctccgggttg ccgccgccgc cgccgcccgc agcccacgtg cggccgctgc tgcgcccgag 1920
   ctcacgcccc gcggccgctt tgttgctccc ggccggcctg cacgatgcac acgccggact 1980
   tcgcaggccc agacgacgcg cgcgcagtga gtggtggggc tgccgcggcg ggactactcg 2040
   tctgagcgag ggcgaggcgg ggggaagtgg tgcggcactc gcgcgcctgt aaatgcggga 2100
   ggtggggcgt gcagggcttc gctgcggccg cgccggtagg ggcctgggcg ggctccggag 2160
   ccgggcgggg cggcggccgc gacgggcgcg cccgggtcgg cgggggcgag gagggggcgt 2220
   gttccccgcg cagctttgtc ttgcgcttcc tgggcggccc cgccccgctg gccccgcggc 2280
   tatttccagc catgacgtca ccccggtcct gtgagccgga cggccgttgg gcgggggagc 2340
   ggctggcggg aacgcggcac gcggccttgg gggcggggca agagctgctg gcgggaacgc 2400
   ggcacggttt tgggggcggg gcacgcggcc gtcgggcggg ttaagagcgg ctagcggtag 2460
   tgccgctcgg gcctgggggc gggcaaggtc taggggccgg ggcaacgacg ggaggcggga 2520
   gcgccgcact gccttggagg cggggcaacg cttggaggcg ggaacgcggc acgcgagcgt 2580
   tgggggccct aagcgtcgcg acctgggggc ttcggggagg tagccgtgga cgtggcagaa 2640
   gcgcgggcct cgtggtgtgc gggcactgtg ggccgggcga gccccttccc gcccgtgtgg 2700
   tgagtcggcc tcggcgcccg gttctgtgac atcacagggg gtttagtggc gcagcctgcg 2760
   ggacagaggc cggggatttg aggtggcctc gtcttgtttg atctcgggga acctcggc 2818

<210> 5
<211> 21
<212> DNA
<213> Artificial Sequence

<220>
<223> Description of Artificial Sequence: Primer

<400> 5
caaagatccc rgaaaggtga c 21

<210> 6
<211> 21
<212> DNA
<213> Artificial Sequence

<220>
<223> Description of Artificial Sequence: Primer

<400> 6
ggggtgctga stgggaagag g 21

<210> 7
<211> 23
<212> DNA

<213> Artificial Sequence

<220>
<223> Description of Artificial Sequence: Primer of
      exon 2 TIEG2 A185G variant

<400> 7
ctcggtgttt gttgctatag act                                              23


<210> 8
<211> 23
<212> DNA
<213> Artificial Sequence

<220>
<223> Description of Artificial Sequence: Primer of
      exon 2 TIEG2 A185G variant

<400> 8
cagggaatct tctcacaagt tct                                              23


<210> 9
<211> 24
<212> DNA
<213> Artificial Sequence

<220>
<223> Description of Artificial Sequence: Primer for the
      sequence of TIEG2 G-1534C variant

<400> 9
gtttaagacg aaagaaccgt gata                                             24


<210> 10
<211> 19
<212> DNA
<213> Artificial Sequence

<220>
<223> Description of Artificial Sequence: Primer for the
      sequence of TIEG2 G-1534C variant

<400> 10
gaacagcaag tgcgaggac                                                   19


<210> 11
<211> 24
<212> DNA
<213> Artificial Sequence

<220>
<223> Description of Artificial Sequence: Primer SNP436

<400> 11
ggaaccatgc tgtttatttt cgtc                                             24

<210> 12
<211> 24
<212> DNA
<213> Artificial Sequence

<220>
<223> Description of Artificial Sequence: Primer SNP436

<400> 12
gtcttcatgc gagcagttta tcac                                                24


<210> 13
<211> 21
<212> DNA
<213> Artificial Sequence

<220>
<223> Description of Artificial Sequence: Primer SNP442

<400> 13
gagacgcgag caaccgaaat a                                                   21


<210> 14
<211> 24
<212> DNA
<213> Artificial Sequence

<220>
<223> Description of Artificial Sequence: Primer SNP442

<400> 14
agaggggacc acgcttatag gaac                                                24


<210> 15
<211> 22
<212> DNA
<213> Artificial Sequence

<220>
<223> Description of Artificial Sequence: Primer SNP453

<400> 15
aaacaaccaa acaaaagcca gg                                                  22


<210> 16
<211> 22
<212> DNA
<213> Artificial Sequence

<220>
<223> Description of Artificial Sequence: Primer SNP453

<400> 16
ggtgtatttt ggttcgcctc cg                                                  22


<210> 17

<211> 23
<212> DNA
<213> Artificial Sequence

<220>
<223> Description of Artificial Sequence: Primer SNP451

<400> 17
gggcacgaat tggctggtgg tgg                                              23


<210> 18
<211> 21
<212> DNA
<213> Artificial Sequence

<220>
<223> Description of Artificial Sequence: Primer SNP451

<400> 18
gcgggactac tcgtctgagc g                                                21


<210> 19
<211> 20
<212> DNA
<213> Artificial Sequence

<220>
<223> Description of Artificial Sequence: Primer SNP444

<400> 19
ttcatatgcg tcttggtcat                                                  20


<210> 20
<211> 19
<212> DNA
<213> Artificial Sequence

<220>
<223> Description of Artificial Sequence: Primer SNP444

<400> 20
cgctcagacg agtagtccc                                                   19


<210> 21
<211> 20
<212> DNA
<213> Artificial Sequence

<220>
<223> Description of Artificial Sequence: Primer SNP417

<400> 21
acaggtgtcc ttgtcgatgg                                                  20


<210> 22
<211> 24

<212> DNA
<213> Artificial Sequence

<220>
<223> Description of Artificial Sequence: Primer SNP417

<400> 22
agaaagcaga tttggagagg gata                                              24


<210> 23
<211> 17
<212> DNA
<213> Artificial Sequence

<220>
<223> Description of Artificial Sequence: Primer SNP305

<400> 23
atctcgtgga gccatcg                                                      17


<210> 24
<211> 20
<212> DNA
<213> Artificial Sequence

<220>
<223> Description of Artificial Sequence: Primer SNP305

<400> 24
acagaaatca gaggggtggt                                                   20


<210> 25
<211> 18
<212> DNA
<213> Artificial Sequence

<220>
<223> Description of Artificial Sequence: Primer SNP306

<400> 25
ggcctggtgc agttcaga                                                     18


<210> 26
<211> 20
<212> DNA
<213> Artificial Sequence

<220>
<223> Description of Artificial Sequence: Primer SNP306

<400> 26
gaagatgggc cttaaggtgg                                                   20


<210> 27
<211> 23
<212> DNA

<213> Artificial Sequence

<220>
<223> Description of Artificial Sequence: Primer SNP416

<400> 27
acgtctactg caatgtgaac aga                                              23


<210> 28
<211> 24
<212> DNA
<213> Artificial Sequence

<220>
<223> Description of Artificial Sequence: Primer SNP416

<400> 28
cctacttcaa aagttcccac ctta                                             24


<210> 29
<211> 22
<212> DNA
<213> Artificial Sequence

<220>
<223> Description of Artificial Sequence: Primer SNP300

<400> 29
tttcctttct ctttaatatg ta                                               22


<210> 30
<211> 17
<212> DNA
<213> Artificial Sequence

<220>
<223> Description of Artificial Sequence: Primer SNP300

<400> 30
gaggaatcca tcagttc                                                     17


<210> 31
<211> 24
<212> DNA
<213> Artificial Sequence

<220>
<223> Description of Artificial Sequence: Primer SNP418

<400> 31
aaaagaaggc tgcaaaatga tcta                                             24


<210> 32
<211> 20
<212> DNA
<213> Artificial Sequence

<220>
<223> Description of Artificial Sequence: Primer SNP418

<400> 32
cctctgcctg aaaggtccat                                                20


<210> 33
<211> 16
<212> DNA
<213> Artificial Sequence

<220>
<223> Description of Artificial Sequence: Primer for
      the sequence of TIEG2 G-1534C variant

<400> 33
tcttcccact cagcac                                                    16


<210> 34
<211> 26
<212> DNA
<213> Artificial Sequence

<220>
<223> Description of Artificial Sequence: Primer for
      the sequence of TIEG2 G-1534C variant

<400> 34
tccctgcgtc cactccagct cccaga                                         26


<210> 35
<211> 18
<212> DNA
<213> Artificial Sequence

<220>
<223> Description of Artificial Sequence: Primer of
      exon 2 TIEG2 A185G variant

<400> 35
atcccagaaa ggtgacct                                                  18


<210> 36
<211> 27
<212> DNA
<213> Artificial Sequence

<220>
<223> Description of Artificial Sequence: Primer of
      exon 2 TIEG2 A185G variant

<400> 36
tcttgtttgt atgagctcct ggggtca                                        27


<210> 37

<211> 402
<212> DNA
<213> Homo sapiens

<220>
<223> Variant G-1534C (SNP437b)

<400> 37
gaacagcaag tgcgaggacc tggagacggc agaggggctg gtgggacagg cgcccaagag 60
gaggcccagg ctgtccctcc ggggcgtggg gagaggcatg gggctgggtc cctgcaggag 120
ctcacaagcc acagtgggga gcagaagcgt tacgctgagt acagtgggag ccactgaaag 180
ggtaaaaacc cctgggggtg ctgastggga agaggctctg ggagctggag tggacgcagg 240
gagaccagtt ataaggcgaa tgcaggagtc caggcaaaag agaatattcc agtgtgtatc 300
ttaagactta acacttattt ttacacacag aacaccctgc gcccctctca gagcctccgg 360
ggtcttcatg cgagcagttt atcacggttc tttcgtctta aa 402

<210> 38
<211> 446
<212> DNA
<213> Homo sapiens

<220>
<223> Sequence of 304a

<400> 38
gaaggaggct ccgtaatcag accttttgtg tggtgagggt ctggaccccc tggggacaaa 60
tcggactttc taaggtgggc aggggctgtg ttttctgagg acatatggca gcctttagtt 120
ttaagagttg ggaaggaaaa gaaggaagag tgaagcctgt ggctccatga tgtactttgc 180
aatcctgggc caggggtgga aggaaataat ggaagacttc caagcacttc ttccagagta 240
gctggaaaac cactttcaat tctacctctt tgcagccacc ccctggcaca ggaagggcac 300
gagcactgac ggccacaggg tcacccggca cactactggg tctgtctgga ccagacccac 360
tgccagagtg gccacttcac atgttggctt tttgagacag cgtcttgctc tgttgcccag 420
gctagggtgc agtggtatga tcatag 446

<210> 39
<211> 1132
<212> DNA
<213> Homo sapiens

<220>
<223> Sequence of 54a (SHGC24187)

<400> 39
aacagcgcgg cttctgctga agccagtaca agcctggtta tctggcttga cccgcccaca 60
cggaggcctc cccagcgttt tctttttccc gagaagagct ccagctcgca ggtccttaga 120
acacggcaga gcaggagcat ctcaggaacc aacggccagc tccttggcgc tgtccccagc 180
cccacaggcc tgctgcggcc atccgcaaac gggcatttcc ttgaactttt gagagtgcct 240
ttgaccagct gttcgcagct gggaaactcc aacttttgaa gtcagagctt ggagcggccc 300
ccgcaagggc gtggctcaaa ccctccccag agctgggagc cattgcttat gcaagcccgt 360
ttcttttgat gtaaagatgg aagtcgttac tatattttaa taatgcaaat acatctttta 420
aaataagtta acatttctta ccaccagatg gatcaggctt tgaatttaat catgtaaatg 480
tttgtaattt tatgtcattt tgttaaaatg ggacgctttc aattggtttc caagaaagat 540
gatacctctg cattttctgg tggaaaaggt gtaataccct taatgagatc aaagtgttag 600
gggaaaaaaa ttccaaaagt agttacaagc tatatcaatg tcaaagtaaa tgcacttcat 660
caaagctaag aagtcacagg aattgttctc aggtttttaa aaaaattttt cctgaattca 720
ggaagtgtct tctgaatagc agctagccaa ataaagcggt gtgtgtgtac tgcagctgta 780
ggtgaactta aaaataataa taaaaagaac aaataaagca gtgtgtacca gccgctgagc 840
cggccctgtc tctgtcctgt gagacggagg ggctgtgcct ggaagtagag gggtgggcgt 900
tgctgcagcc tgcagcccgg cccgagtctg tcccagtgcc tcctgtgtgc ctggcacctc 960

ctcaggcatc ttcctggctg ccccatgttg cagctgagcc tcagcgtcct gctagaactg 1020
cgtgtgggat ttgctcactg ccaggatggg actgtggctg gaatggaggc agaaagaaac 1080
acatctcaaa ggcaaaaatg acgcacgttg ttgtctggaa aaatcttaag tg 1132

## Description

The invention pertains to the field of human genetics and relates to new methods of diagnosis and therapy of type 2 diabetes, based on the identification of the A185G variation located at exon 2 of the *tieg2* gene as well as the G-1534C, 54a and 304a variations.

Type 2 diabetes, also known as noninsulin dependent diabetes mellitus (NIDDM) or adult onset diabetes, is the most common form of diabetes. In type 2 diabetes, the pancreatic cells produce little or no insulin, or the cells throughout the body are unable to utilize the insulin (insulin resistance). It represents 95% of all cases in the western countries, which affects about 15 million people in the United States alone.

As of today, the fasting plasma glucose (FPG) test is employed for diagnosing diabetes. However, this test is only relevant once the disease has developed. Therefore, there is a need for a diagnostic test capable of evaluating the genetic risk factor associated with this disease. Such a test would be of great interest in order to adapt the lifestyle of people at risk and to prevent the onset of the disease.

There is strong evidence to support the involvement of genetic factors in the genesis of type 2 diabetes. For example, the risk rate for type 2 diabetes between identical twins is around 60 to 90 percent (Bennett PH. Epidemiology of diabetes mellitus. In: Rifkin H, Porte D Jr., eds. Ellenburg and Rifkin's Diabetes Mellitus. New York: Elsevier, 1990:363-77). In addition, having one first-degree relative with type 2 diabetes doubles the risk of developing the disease (Leslie RD, Pyke DA. Genetics of diabetes. In: Alberti KG, Krall LP, eds. The diabetes annual. Amsterdam: Elsevier, 1987:39-54).

More recently, numerous scientific studies have established that there is a genetic predisposition to type 2 diabetes. We have performed a genome wide scan of diabetic and obese families, which led to the identification of several genomic regions showing evidence for linkage to these diseases (Vionnet N et al. (2000):
Genomewide search for type 2 diabetes susceptibility genes in French whites: Evidence for a novel susceptibility locus for early onset diabetes on chromosome 3q27 qter and independent replication of a type 2 diabetes locus on chromosome 1q21 q24. Am. J. Hum. Genet. 67 : 1470 1480 and Hager J et al. (1998): A genome wide scan for human obesity genes reveals a major susceptibility locus on chromosome 10. Nat. Genet. 20 :304 308.).

Further fine mapping with microsatellite markers allowed us to limit this region to approximately 5 Mbases, which makes it feasible to identify candidate genes and apply a LD mapping strategy for this region. LD mapping involves the genotyping of a vast amount of single nucleotide polymorphisms (SNPs).

Over the years, we have developed complete strategies and methodologies to identify positional gene candidates. These include statistical analysis, a bioinformatic pipeline, SNP genotyping and tissue expression profiling. Our approach has resulted in fine mapping of the chr2 interval and analysis of this genomic region by a positional cloning approach. The SNP map established for chromosome 2 region has lead to the identification of TIEG2 as gene related to susceptibility to type 2 diabetes.

Both TIEG genes are induced by TGF-β (Cook et al. 1998; Blok et al. 1995) and may control pancreatic cell growth (Cook et al. 1998; Cook and Urrutia 2000). TIEG1 over expression in the exocrine PANC1 cell line induced apoptosis (Tachibana et al. 1997) and a similar proliferation inhibiting effect was shown for TIEG2 (Cook et al. 1998). Transgenic mice that express TIEG2 in the acinar cells of the exocrine pancreas (Mladek et al. 1999) show a similar pancreatic atrophy as mice over expressing TGF- β (Sanvito et al. 1995). Moreover, TIEG has been shown to induce similar effects as TGF-β (in osteoblast cells (Hefferan et al. 2000). Thus, TGF-β induced TIEG 1 and 2 are able to mimic effects of TGF-β, in particular those effects observed on exocrine pancreatic tissues.

In connection with the invention, we identified and analyzed the variant A185G located at exon 2 of TIEG2 (=2SNP199b, TG2Q62R, TG2AI85G) and we have shown that TIEG2 is related to susceptibility to type 2 diabetes. The SNP199b (A>G) is a non synonymous (Gln62Arg) variation in exon 2 of TIEG2, which can alter the repressive properties of TIEG2 on transcription.
In connection with the invention, we identified and analyzed an additional variant, G-1534C, which is in close disequilibrium with A185G. This variant is located 1.5 Kb from the translation start site and may reside in the yet undefined promotor of the TIEG2 gene. Analysing this sequence with TransFac (Heinemeyer et al 1998) predicted that the introduction of the C allele creates a AP-1 site. The presence of both the -1534C and 185G allele could result in overexpression of a non-functional TIEG2 protein.

In this regards, altered regulation of progenitor cells in the developing pancreas and/or altered neogenesis of β cells in the adult human pancreas, which might be caused by a less functional TIEG2 regulation, may change the balance between exocrine and endocrine cells.

TIEG2 is implicated in the endocrine exocrine balance of the pancreas, and is postulated here to be important in the development and regeneration of pancreatic tissues, which could influence the development of type 2 diabetes. TIEG2 is ubiquitous expression, but has elevated mRNA levels in skeletal muscle, exocrine pancreas, brain and adipose tissue, which suggest that TIEG2 may have additional roles, which could involve proliferation/differentiation and cell cycle regulation, that could influence susceptibility to type 2 diabetes in several tissues.

### Description

Therefore, the present invention contributes to a better handling of type 2 diabetes and provides evidence of the implication of TIEG2 mediated signaling in susceptibility for type 2 diabetes. As a result, TIEG2 represents a novel target for drug design for Type 2 diabetes. The association of TIEG2 variant TG2Q62R with type 2 diabetes is particularly useful as a diagnostic test to analyze predisposition to type 2 diabetes.

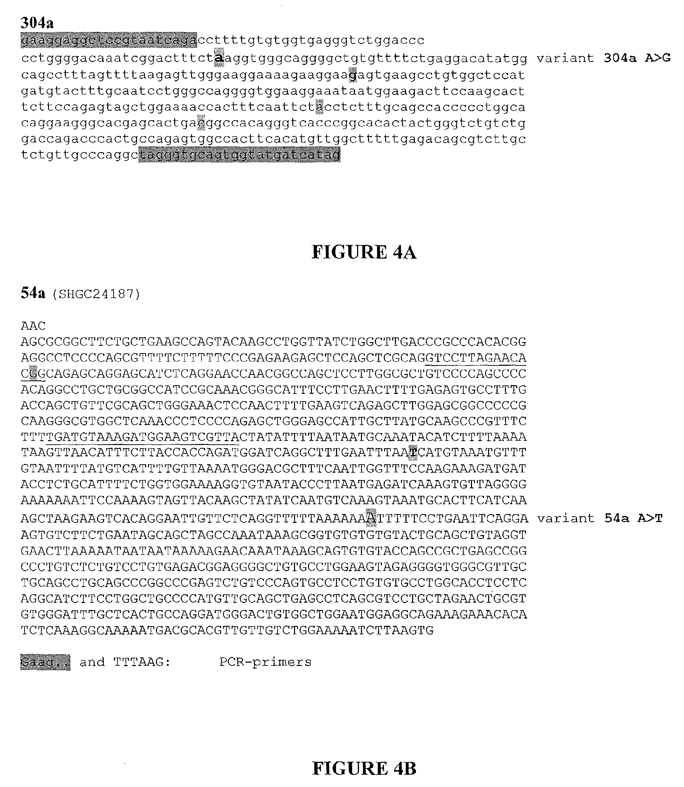

In a first aspect, the invention relates to a method for diagnosing a predisposition for type 2 diabetes in a human subject which comprises determining whether there is a germline variation in the sequence of the *tieg2* gene, said variation being indicative of a predisposition to type 2 diabetes, wherein said variation is selected from A185G numbered with regards to the start codon of the *tieg2* gene, the coding sequence of which is represented by SEQ ID N° I, G-1534C as shown in SEQ ID N° 4, 54a or 304a as shown in figure 4A and 4B.

It also relates to a method for diagnosing a predisposition for type 2 diabetes as depicted above comprising determination of the presence of germline variations that are in close association with a variation selected from variation A185G, G-1534C, 54a or 304a.

The invention relates more generally to the study of the *tieg2* gene.
In this regard, the invention relates to a method for identifying variations in the sequence of the *tieg2* gene, comprising determining whether there is a mismatch between molecules (1) a region *tieg2* gene genomic DNA isolated from said sample, and (2) a nucleic acid probe complementary to human wild-type region of the *tieg2* gene DNA, when molecules (1) and (2) are hybridized to each other to form a duplex, and said mismatch being due to the presence of at least one variation in the sequence of the *tieg2* gene genomic DNA isolated from said sample.

More specifically, the invention is aimed at a method for identifying variations in exon 2 of the *tieg2* gene comprising determining whether there is a mismatch between molecules (1) exon 2 of the *tieg2* gene genomic DNA isolated from said sample, and (2) a nucleic acid probe complementary to human wild-type exon 2 region of the *tieg2* gene DNA as represented by SEQ ID N° 2, when molecules (1) and (2) are hybridized to each other to form a duplex, and said mismatch being due to the presence of at least one variation in the sequence of the *tieg2* gene genomic DNA isolated from said sample.

In the above mentioned method, mRNA of the sample is contacted with a *tieg2* gene probe under conditions suitable for hybridization of said probe to a RNA corresponding to said *tieg2* gene and hybridization of said probe is determined, and the level of signal after hybridization is compared with a standard signal (which is either a positive control, a negative control or both).

The hybridization complex emits a signal, which may be due to the labeling of the probe, or directly of the mRNA. The different labels that may be used are well known to the person skilled in the art, and one can cite ³²P, ³³P, ³⁵S, ³H or ¹²⁵I. Non radioactive labels may be selected from ligants as biotin, avidin, streptavidin, dioxygenin, haptens, dyes, luminescent agents like radioluminescent, chemoluminescent, bioluminescent, fluorescent or phosphorescent agents.
In order to identify the SNPs mentioned in the present application, it is possible to contact a *tieg2* gene probe with genomic DNA isolated from said sample under conditions suitable for hybridization of said probe to said gene and hybridization of said probe is determined.

Said *tieg2* gene region probe is either a "wild-type" DNA, (i.e. the searched SNP is not present on the probe, and hybridization occurs when no SNP according to the invention is present on the DNA in the sample) or a "mutant" one (i.e. carrying the searched SNP, and hybridization only occurs if the SNP is present on the DNA in the sample).
The person skilled in the art knows the techniques to determine the allele specific probes to use in this embodiment, their lengths, or the hybridization conditions.

In another embodiment, the invention is performed by determining whether there is an alteration in the germline sequence of the *tieg2* gene exon 2 region or other regions in said sample by observing shifts in electrophoretic mobility of single-stranded DNA from said sample on denaturing or non-denaturing polyacrylamide gels. Said single-stranded nucleic acids may be obtained after amplification of the genomic DNA, using suitable primers, and denaturation (the gel and electrophoresis conditions are usually denaturing for such a purpose).

In another embodiment, the invention is performed by amplification of all or part of the *tieg2* gene exon 2 region or other regions from said sample, and determination of the sequence of said amplified DNA.

In another embodiment, allele specific oligonucleotide primers are employed to determine whether a specific *tieg2* mutant allele is present in said sample, the amplification only occurring in this case.

These primers cover the sequences -caaagatccc**R**gaaaggtgac- (SEQ ID No 5) and -ggggtgctga**S**tgggaagagg- (SEQ ID No 6) for, respectively, A185G and G-1534C, wherein R designates A or G and wherein S designates C or G.

In another embodiment, all or part of the *tieg2* gene exon 2 region from said sample is cloned to produce a cloned sequence and the sequence of said cloned sequence is determined.

The method depicted above can be practiced by amplification of *tieg2* gene exon 2 region sequences in said sample and hybridization of the amplified sequences to one or more nucleic acid probes issued from the wild-type *tieg2* gene exon 2 region sequence (as represented in SEQ ID N° 2) or a mutant *tieg2* gene exon 2 region sequence, said probes being selected from probes specific for the A185G variation and the G-1534C variation.

The A185G variation assay can be performed by a PCR with oligonucleotide primers to amplify the genomic sequence of exon 2 TIEG2 5'-CTC GGT GTT TGT TGC TAT AGA CT-3' (SEQ ID N° 7) and 5'-CAG GGA ATC TTC TCA CAA GTT CT-3' (SEQ ID N° 8), Table 1), whereafter the allelic variation can be measured by differential Tm changes using a lightcycler (Roche) with the probes LCRed640-ATC CCA GAA AGG TGA CCT (SEQ ID N° 33) and TCT TGT TTG TAT GAG CTC CTG GGG TCA-fluorescine (SEQ ID N° 34).

The G-1534C variation assay can be performed by a PCR with oligonucleotide primers to amplify the genomic sequence upstream of TIEG2 (5'-GTT TAA GAC GAA AGA ACC GTG ATA-3' (SEQ ID N° 9) and 5'-GAA CAG CAA GTG CGA GGA C-3', (SEQ ID N° 10), Table 1), whereafter the allelic variation can be measured by differential Tm changes using a lightcycler (Roche) with the probes LCRed640-TCTTCCCACTCAGCAC (SEQ ID N° 35)
and TCCCTGCGTCCACTCCAGCTCCCAGA-fluorescine (SEQ ID N° 36).

The fluorescent molecules attached to the probes are shown as examples and are not limiting.

In still another embodiment, the invention is aimed at a probes or a primer selected from SEQ ID N°5 to SEQ ID N°36 (see Tables 1 hereinafter).

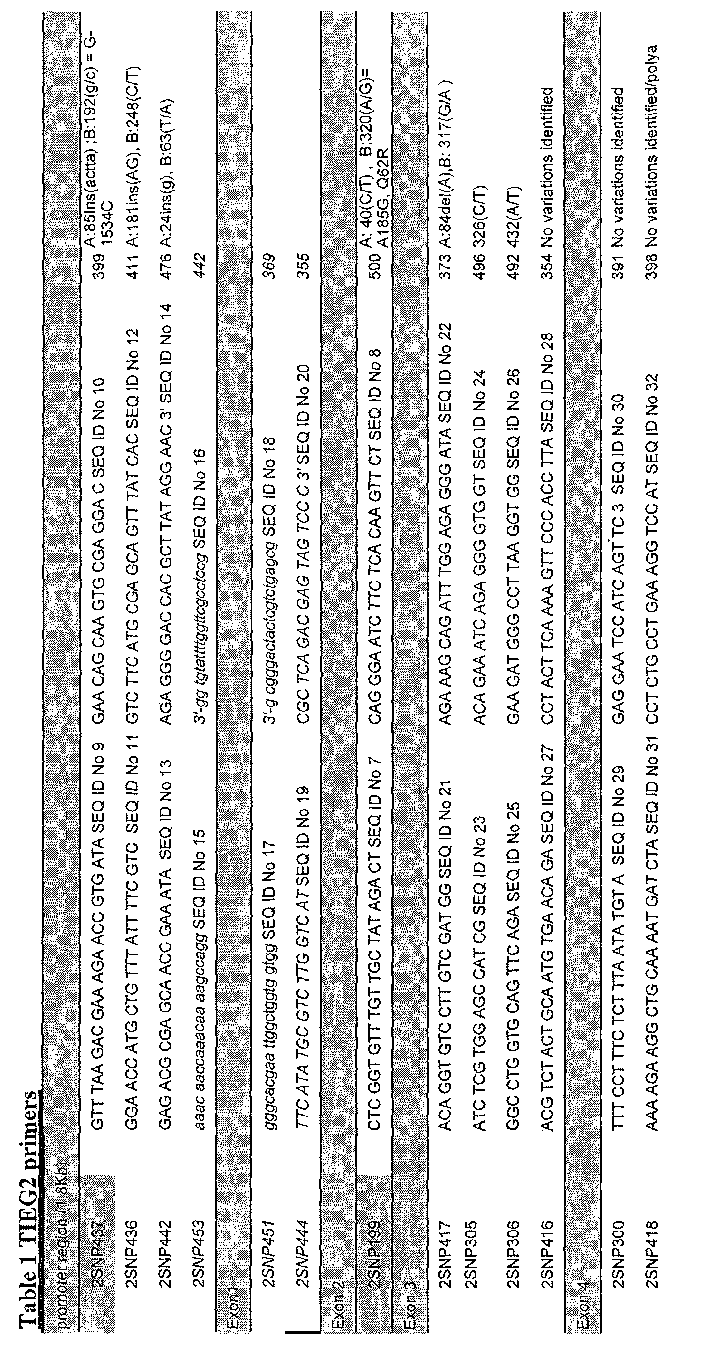

The invention is also directed to the fragments of SEQ ID No 1 and SEQ ID No 4 amplified with the probes listed in table 1 and diagnostic kits comprising at least one of the identified variants.

The invention also relates to a method which comprises determining in *situ* hybridization of the *tieg2* gene exon 2 region in said sample with one or more nucleic acid probes issued from a wild-type or a mutant *tieg2* gene exon 2 region sequence.

The inventors have demonstrated that the presence of the GIn62Arg variation of TIEG2 may interfere with the TIEG2 transcriptional repression activity in several ways. It is thus credible to speculate that other modifications in the exon 2 region or in other regions of the *tieg2* gene, leading to a modification of the transcriptional repression activity of TIEG2 protein, will also lead to predisposition to type 2 diabetes, due to an alteration of the interaction with mSin3A.

In general, shown for the first time for Mad proteins, interaction with the corepressor mSin3A evokes recrutement of histone deacetylases and the formation of a functional transcription repressor complex. Recently it has been shown that phosphorylation nearby the first repressor domain disrupts the mSin3A binding to TIEG2 and results in loss of TIEG2's repressor activity (Ellenrieder et al 2002). Similar, occurence of the 62R variant may interfere with mSin3A binding changing the repressor activity of TIEG2. In addition, occurence of the 185G variant with the - 1534C (%) variant, which introduces a possible transcriptional upregulation of TIEG2 via introduction of an AP-1 response site in TIEG2's promoter, could result in overexpression of a non-functional TIEG2 protein.

Thus, in one embodiment, the invention relates to a method for diagnosing a predisposition for type 2 diabetes in a human subject, wherein the alteration of the interaction of TIEG2 protein with mSin3A and expression levels of TIEG2 mRNA in said sample is investigated. Alteration of interaction between protein and nucleic acid can be detected by any techniques known in the art.

In a second aspect, the invention relates to the GIn62Arg variant of TIEG2 of SEQ ID n°3. In particular, this polypeptide may be detected by immunoblotting or immunocytochemistry.

Thus, the invention is aimed at antibodies specific for this TIEG2 variant, i-e antibodies capable of discriminating between normal TIEG2 and variant TIEG2, can be used to detect the presence or the absence of said variant in the sample of a patient. Immunological assays can be performed using standard knowledge in the art. These include Western blots, immunohistochemical assays and ELISA assays. The invention encompasses a diagnostic kit comprising such an antibody and reagents suitable for these tests.

The invention opens up a new area in the treatment and/or prevention of type 2 diabetes, especially for patient in families where genetic susceptibility is suspected.
Thus, the invention also relates to a method for screening compounds capable of preventing and/or treating type 2 diabetes which comprises: combining (i) a candidate compound and (ii) a TIEG2 polypeptide and determining the amount of binding of the TIEG2 polypeptide to said compound. Indeed, agonists of TIEG2 will reinstate its transcriptional repression activity and can be considered as useful drugs for treating type 2 diabetes, alone or in association with other treatments.

In this embodiment, the term "TIEG2" must be understood as including the GIn62Arg variant as well as wild-type TIEG2.

The invention also relates to a pharmaceutical composition comprising a pharmaceutically acceptable excipient and a compound identified by a method according to the invention, and to the use of a compound identified by a method according to the invention for the manufacture of a drug intended to treat and/or prevent type 2 diabetes.

The invention also relates to a method for the therapy of type 2 diabetes comprising administration of the pharmaceutical composition of the invention.

In order to correct the defect due to the GIn62Arg variant, another embodiment of the invention relates to a vector, more particularly a vector suitable for gene therapy, comprising the coding sequence for the wild-type TIEG2 operatively linked to a promoter allowing the expression of said coding sequence in human cells. Such vectors can be adapted for direct in vivo gene transfer and include viral vectors, such as adenovirus, retrovirus, herpes virus vectors, and adeno-associated virus (AAV). Nonviral vectors may also be used, including liposomes, molecular conjugates, polymers and other vectors. Direct injection into tissues, intravenous or intra-arterial administration, inhalation, or topical application and other routes of administration are known in the art.

The invention also relates to new animal models useful for studying type 2 diabetes, wherein said *tieg2* variant is expressed in said animal. The methods to obtain animals according to the invention are known by the persons skilled in the art, and are useful in particular for testing some drugs that can be identified according to the methods of the invention. The methods include genetically modifying animals to provide a new line capable of germinal transmission of said *tieg2* variant gene, chimeric animals, and animals which have been transfected with a vector expressing said *tieg2* variant.

The insertion of the construct in the genome of the transgenic animal of the invention may be performed by methods well known by the artisan in the art, and can be either random or targeted. In a few words, the person skilled in the art will construct a vector containing the sequence to insert within the genome, and a selection marker (for example the gene coding for the protein that gives resistance to neomycine), and may have it enter in the Embryonic Stem (ES) cells of an animal. The cells are then selected with the selection marker, and incorporated into an embryo, for example by microinjection into a blastocyst, that can be harvested by perfusing the uterus of pregnant females. Reimplantation of the embryo and selection of the transformed animals, followed by potential back-crossing allow to obtain such transgenic animal. To obtain a "cleaner" animal, the selection marker gene may be excised by use of a site-specific recombinase, if flanked by the correct sequences.

The invention thus relates to a transgenic non-human mammal having integrated into its genome the nucleic acid sequence of the *tieg2* variant gene or the coding sequence thereof, operatively linked to regulatory elements, leading to the expression of the TIEG2 GIn62Arg variant protein.

The *tieg2* sequence foreseen for the preceding embodiment may be a human *tieg2* gene sequence introduced within the genome of the animal of the invention, or the endogenous *tieg2* gene sequence the promoter of which has been modified in order to induce overexpression, so long as the variation is present in the sequence.

Furthermore, the invention also relates to a transgenic non-human mammal whose genome comprises a disruption of the endogenous *tieg2* gene, as an animal model for testing links bewteen TIEG2 and type 2 diabetes. In particular, said disruption comprises the insertion of a selectable marker sequence. In particular, said disruption is a homozygous disruption, and said homozygous disruption results in a null mutation of the endogenous gene encoding TIEG2.
The mammal of the invention is preferably a rodent such as a mouse.

### Legends

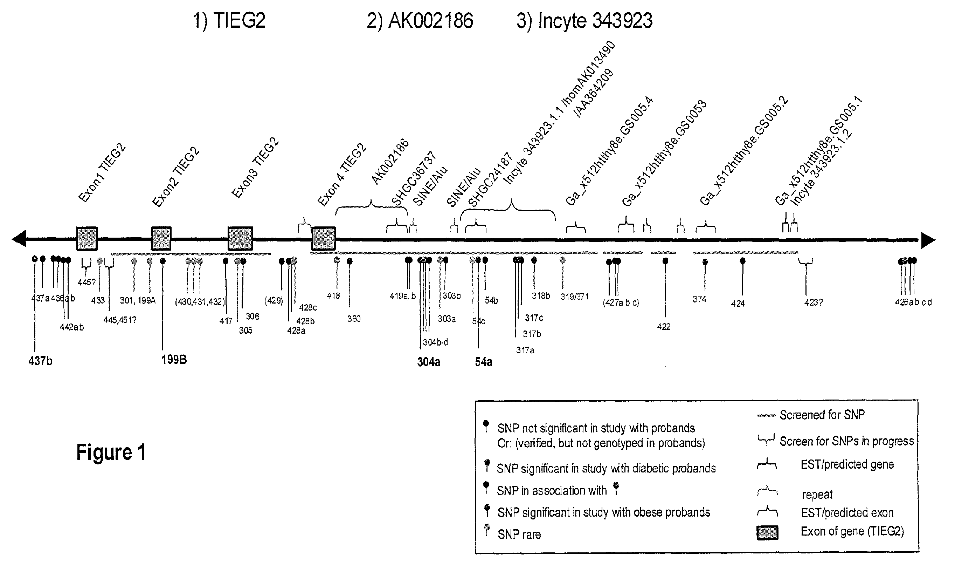

### FIGURE 1: SNP map and contig close to TIEG

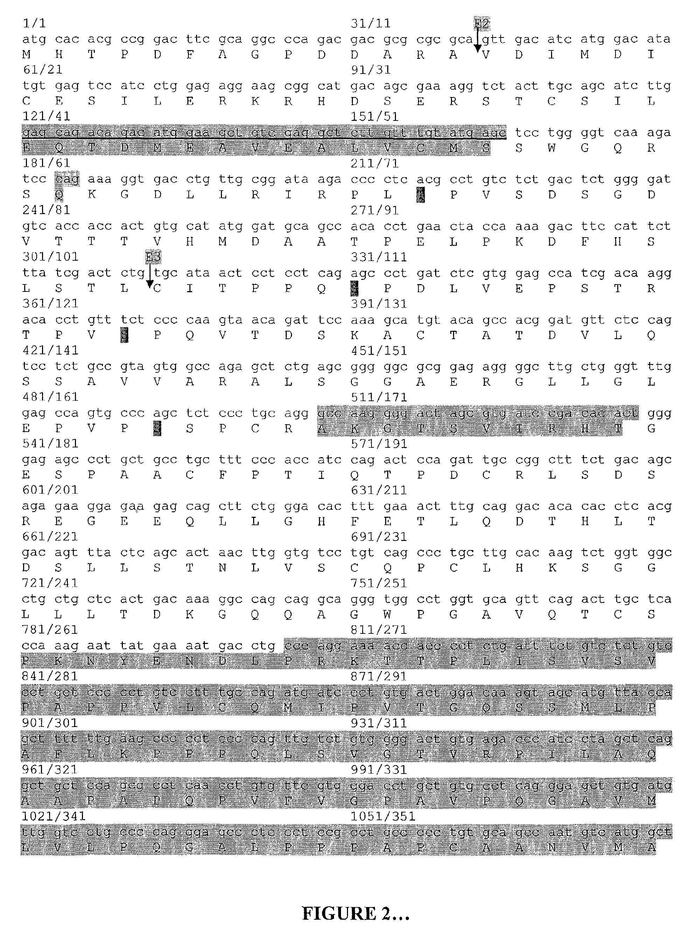

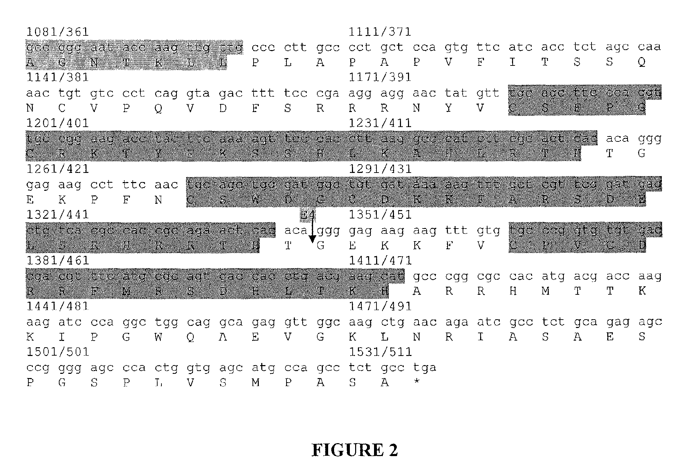

### FIGURE 2: illustrated TIEG2 CDS

E1-4:        exon 1 to 4
Atg:         start codon
S/T:         reported phosphorylation sites
Gag..:       repressor domains
 Gag..:      repressor domain with interaction mSin3A

Tgc..:       Zn-finger domains

Cag:         A185(G)
Q            Gln62 (Arg)

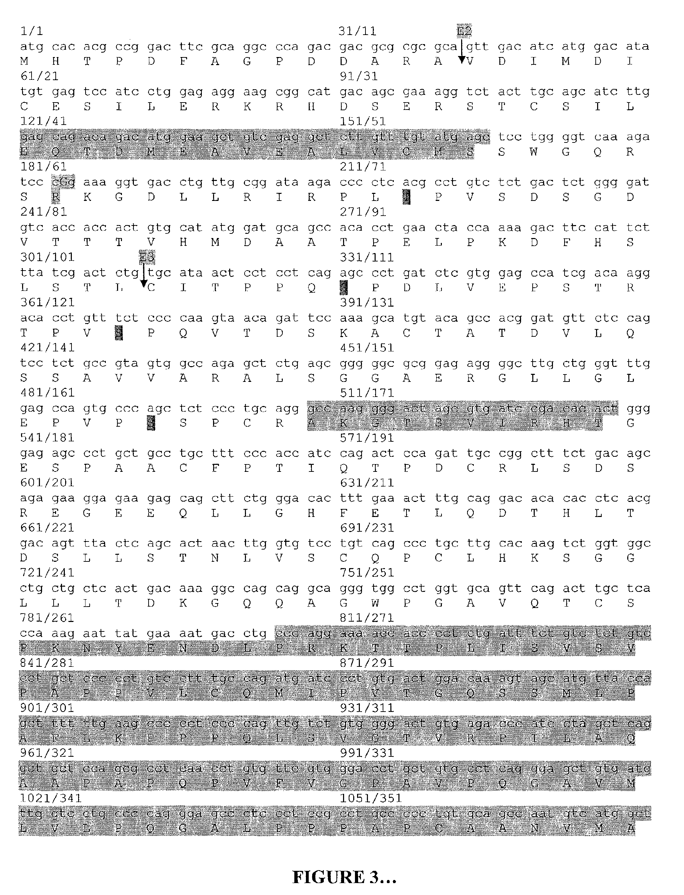

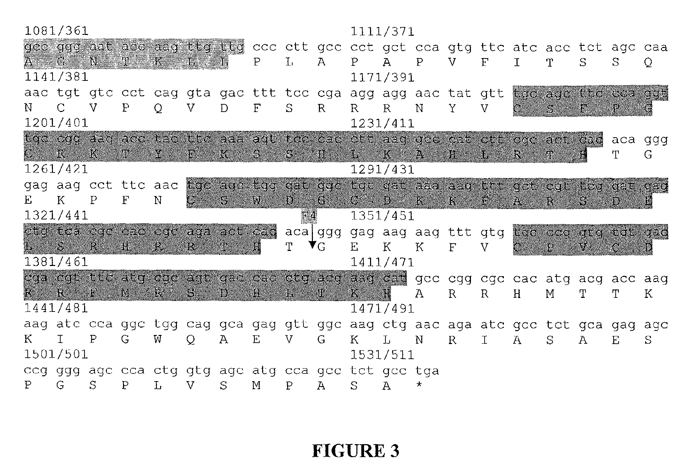

### FIGURE 3: illustrated tieg2mtQ62R

Legend:
E1-4:      exon 1 to 4

Atg:          start codon
S/T:          reported phosphorylation sites
Gag..:        repressor domains
Tgc..:        Zn-finger domains

CGg:          (A) 185G
R             (Gln)62Arg

### FIGURE 4: illustrated Sequence of 304a and 54a

### EXAMPLE 1: Genetic evidence for implication of TIEG2 in type 2 Diabetes (T2D)

SNP199b (A>G) is a exonic variation of TIEG2. A robust association of SNP 199b (TG2Q62R) with T2D was shown to exist in a French Caucasian cohort of familial T2D and controls (Logistic regression (stratified for aga and sex) under dominant model TG2Q62R p<10-5). SNP 199b, 437b, 54a and 304a are in strong association (chi-square p<0.0001). SNP 437b (G>C) is a genomic variation that may reside in the yet undefined promotor of the TIEG2 gene. We have shown association of 54a with diabetes in all members of the genome wide scan families showing linkage evidence to chr 2 (phenotype large/strict, xtd lod-score 4.45 (p=0.04)). In contrast to the dominant association with familial type 2 diabetes (in the cohort of 352 controls vs 287 diabetics), no dominant association to diabetes was identified in the more heterogeneous 'Corbeil' population (947 diabetics). However, the variances remained recessively associated with a higher BMI.

The Gln62Arg is located in the proximity of the first repressor domain of the TIEG2-protein, which has been shown to interact with mSin3A corepressor. It has been shown that the R1 domain of TIEG2 adopts an a-helix conformation, and that this a -helical repression motif (oc-HRM), which is conserved in TIEG 1, BTEB1, 3 and 4, is responsible for the interaction with mSin3A (Sin Interacting Domain; (Zhang et al. 2001)). Analyses of the secondary protein structure of TIEG2 (62G1n) and 62Arg-TIEG2 (protein containing TG2Q62R) with PIX (UK HGMP resource centre http://www.hizmp.mrc.ac.uk~ showed that DSC (one out of three 2D analysing programs used (King et al. 2000)) predicted a prolonged a-helix for the 62Arg-TIEG2 in proximity of the SID. Thus, an altered conformation of 62Arg-TIEG2, may interfere with the a -HRM-mediated interaction with mSin3A.

In addition, the glutamine to arginine-change adds extra charge to the protein and may, therefore, change its isoelectric point and its binding properties to mSin3A.

Furthermore, Gln62Arg is next to a serine, which may be phosphorylated by Protein kinase C. Phosphorylation can play a crucial role in either regulating protein activity, translocation to the nucleus or interaction with other proteins and sensitivity to proteolytic degradation.

G-1534C is located 1.5 Kb from the translation start site and may reside in the yet undefined promotor of the TIEG2 gene. Analysing this sequence with TransFac (Heinemeyer et al 1998) predicted that the introduction of the C allele creates an AP-1 site. The presence of both the -1534C and 185G allele (linkage disequilibrium 0.95) could result in overexpression of a non-functional TIEG2 protein.

In summary, although TIEG2 has been implicated in the proliferation of exocrine pancreatic cells, analyses of the newly identified TG2Q62R (SNP199b) has, for the first time, directly implicated an altered TIEG2 in susceptibility for Type 2 diabetes. Furthermore, we hypothyse that since TIEG2 is ubiquitous expressed but higher expression levels are found in skeletal muscle, exocrine pancreas, brain and adypocytes, TIEG2 may affect susceptibility to T2D by its effect on these tissues. The association of TG2Q62R (SNP199b) with type 2 diabetes is useful as a diagnostic test to analyse predisposition to type 2 diabetes.

To further investigate the role of TIEG2 in the development of type 2 diabetes, we aimed at identifying target genes of TIEG2. First, we have concentrated on genes playing a major role in the pancreas. Based on the reported SPl-like binding site for TIEG2 (Cook et al 1998), we expect TIEG2 binding elements in the promoters of Smad4, IGF2 and PDX-1. For PDX-1 this binding element may reside in the recently characterised <Enhancer 1> element (Ben-Shushan et al 2001). Indeed, we have shown that a TIEG2 expression vector (kind gift of Dr. R. Urrutia) stimulates the activity of the PDX-1 enhancer element severeal fold, whereas it had no effects on the mutated enhancer element **(figure..).** PDX-1 is critical for development of the pancreas and regulates transcription of genes crucial for proper islet cell function, e.g. insulin gene (reviewed by Hui et al 2002). Moreover, mutations of the PDX-1 gene are held responsible for the development of maturity onset diabetes of the young type 4 (MODY 4). Thus, regulation of PDX-1 by TIEG2 shows that inproper function of TIEG2 may contribute to the development of type 2 diabetes. We are curently investigating the exact mechanism of PDX-1 gene regulation by TIEG2. On one hand, this may occur via direct interaction with the Enhancer 1 element. On the other hand, TIEG2 may affect PDX-1 gene expression via regulation of smad signalling since it was recently reported that the closely related TIEG1 regulate both smad7 and smad2 gene expression (Johnsen et al 2002a, b).

Therefore, we conclude that the G-1534C and Gln62Arg variation of TIEG2 may interfere with TIEG2 transcriptional activity in several ways, and consequently may have a causal association with the development of type 2 diabetes.

### REFERENCES

Ben-Shushan E, Marshak S, Shoshkes M, Cerasi E, Melloul D. (2001) A pancreatic beta -cell-specific enhancer in the human PDX-1 gene is regulated by hepatocyte nuclear factor 3beta (HNF-3beta ), HNF-lalpha, and SPs transcription factors. J. Biol. Chem. 276,17533-17540.

Blok LJ, Grossmann ME, Perry JE, Tindall DJ (1995) Characterization of an early growth response gene, which encodes a zinc finger transcription factor, potentially involved in cell cycle regulation. Mol Endocrinol 9:1610-20.

Cook T, Gebelein B, Mesa K, Mladek A, Urrutia R (1998) Molecular cloning and characterization of TIEG2 reveals a new subfamily of transforming growth factor-beta-inducible Spl-like zinc finger- encoding genes tnvolved in the regulation of cell growth. JBiol Chem 273:25929-36.

Cook T, Urrutia R (2000) TIEG proteins join the Smads as TGF-beta-regulated transcription factors that control pancreatic cell growth. Am JPhysiol Gastrointest Liver Physiol 278: 6513-21.

Ellenrieder V, Zhang JS, Kaczynski J, Urrutia R.(2002) Signaling disrupts mSin3A binding to the Madl-like Sin3-interacting domain of TIEG2, an Sp1-like repressor. EMBO J. 21,2451-2460.

Hefferan TE, Reinholz GG, Rickard DJ, Johnsen SA, Waters KM, Subramaniam M, Spelsberg TC (2000) Overexpression of a nuclear protein, TIEG, mimics transforming growth factor-beta action in human osteoblast cells. J Biol Chem 2 75:20255-9.

Heinemeyer, T., Wingender, E., Reuter, I., Hermjakob, H., Kel, A. E., Kel, O. V., Ignatieva, E. V., Ananko, E. A., Podkolodnaya, O. A., Kolpakov, F. A., Podkolodny N. L. and Kolchanov, N. A.(1998) Databases on Transcriptional Regulation: TRANSFAC, TRRD, and COMPEL. Nucleic Acids Res. 26, 364-370

Hui H, Perfetti R.(2002) Pancreas duodenum homeobox-1 regulates pancreas development during embryogenesis and islet cell function in adulthood. Eur. J. Endocrinol. 146,129-141.

Johnsen SA, Subramaniam M, Janknecht R, Spelsberg TC. (2002a) TGFbeta inducible early gene enhances TGFbeta/Smad-dependent transcriptional responses. Oncogene 21, 5783-5790.

Johnsen SA, Subramaniam M, Katagiri T, Janknecht R, Spelsberg TC. (2002b) Transcriptional regulation of Smad2 is required for enhancement of TGFbeta/Smad signaling by TGFbeta inducible early gene. J .Cell Biochem. 87, 233-241.

Mladek A, Burgart JL, Urrutia R (1999) Morphological changes in the exocrine pancreas of transgenic mice overexpressing the TGFfi-inducible zinc finger transcription factor TIEG2 in an acinar-specific manner (abstract). Gastroenterology 116:G4837.

Sanvito F, Nichols A, Herrera PL, Huarte J, Wohlwend A, Vassalli JD, Orci L (1995) TGF-beta I overexpression in murine pancreas induces chronic pancreatitis and, together with TNF-alpha, triggers insulindependent diabetes. Biochem Biophys Res Commun 217:1279-86.

Tachibana 1, Imoto M, Adjei PN, Gores GJ, Subramaniam M, Spelsberg TC, Urrutia R (1997) Overexpression of the TGFbeta-regulated zinc finger encoding gene, TIEG, induces apoptosis in pancreatic epithelial cells. J Clin Invest 99:2365-74.

## Claims

1. A method for diagnosing a predisposition for type 2 diabetes in a human subject which comprises determining whether there is a germline variation in the sequence of the *tieg2* gene, said variation being indicative of a predisposition to type 2 diabetes, wherein said variation is selected from A185G numbered with regards to the start codon of the *tieg2* gene, the coding sequence of which is represented by SEQ ID N° 1, G-1534C as shown in SEQ ID N° 4, 54a or 304a as shown in figure 4A and 4B.

2. A method according to claim 1 comprising determination of the presence of germline variations that are in close association with a variation selected from variation Al 85G, G-1534C, 54a or 304a.

3. A method for identifying variations in the sequence of the *tieg2* gene, comprising determining whether there is a mismatch between molecules (1) a region *tieg2* gene genomic DNA isolated from said sample, and (2) a nucleic acid probe complementary to human wild-type region of the *tieg2* gene DNA, when molecules (1) and (2) are hybridized to each other to form a duplex, and said mismatch being due to the presence of at least one variation in the sequence of the *tieg2* gene genomic DNA isolated from said sample.

4. A method for identifying variations in exon 2 of the *tieg2* gene comprising determining whether there is a mismatch between molecules (1) exon 2 of the *tieg2* gene genomic DNA isolated from said sample, and (2) a nucleic acid probe complementary to human wild-type exon 2 region of the *tieg2* gene DNA as represented by SEQ ID N° 2, when molecules (1) and (2) are hybridized to each other to form a duplex, and said mismatch being due to the presence of at least one variation in the sequence of the *tieg2* gene genomic DNA isolated from said sample.

5. A method according to claim 4 comprising the amplification of *tieg2* gene exon 2 region sequences in said sample and hybridization of the amplified sequences to one or more nucleic acid probes issued from the wild-type *tieg2* gene exon 2 region sequence (as represented in SEQ ID N° 2) or a mutant *tieg2* gene exon 2 region sequence, said probes or primers covering the sequences -caaagatcccRgaaaggtgac-(SEQ ID No 5) and -ggggtgctgaStgggaagagg- (SEQ ID No 6) for the TIEG2 variants A185G and G-1534C, respectively, wherein R designates A or G and wherein S designates C or G.

6. A probes or a primer selected from SEQ ID N°5 to SEQ ID N°36.

7. The GIn62Arg protein variant of TIEG2 of SEQ ID n°3.

8. An antibody capable of discriminating between normal TIEG2 and the variant TIEG2 according to claim 7.

9. A diagnostic kit comprising an antibody according to claim 7 and reagents suitable for Western blots, immunohistochemical assays and ELISA assays or at least one probe according to claim 6.

10. A method for screening compounds capable of preventing and/or treating type 2 diabetes which comprises: combining (i) a candidate compound and (ii) a TIEG2 polypeptide and determining the amount of binding of the TIEG2 polypeptide to said compound.

11. A vector suitable for gene therapy, comprising the coding sequence for the wild-type TIEG2 operatively linked to a promoter allowing the expression of said coding sequence in human cells.

12. Use of a compound identified by the method of claim 10 or of a vector according to claim 11 to manufacture a medicament for preventing and/or treating type 2 diabetes.

13. An animal model useful for studying type 2 diabetes, wherein the protein variant of claim 7 is expressed in said animal.

14. A transgenic non-human mammal having integrated into its genome a nucleic acid sequence coding for protein variant of claim 7, operatively linked to regulatory elements, leading to the expression of the TIEG2 GIn62Arg variant protein.

15. A transgenic non-human mammal whose genome comprises a disruption of the endogenous *tieg2* gene, as an animal model for type 2 diabetes.
